(19) Europäisches Patentamt
European Patent Office
Office européen des brevets

(11) Veröffentlichungsnummer: **0 194 620**
**A2**

# (12) EUROPÄISCHE PATENTANMELDUNG

(21) Anmeldenummer: **86103109.4**

(22) Anmeldetag: **08.03.86**

(51) Int. Cl.⁴: **C 07 C 57/04**
C 07 C 51/25

(30) Priorität: **12.03.85 DE 3508702**

(43) Veröffentlichungstag der Anmeldung:
**17.09.86 Patentblatt 86/38**

(84) Benannte Vertragsstaaten:
**BE DE FR GB**

(71) Anmelder: **BASF Aktiengesellschaft**
**Carl-Bosch-Strasse 38**
**D-6700 Ludwigshafen(DE)**

(72) Erfinder: **Bassler, Peter, Dr.**
**Franconvillestrasse 10**
**D-6806 Viernheim(DE)**

(72) Erfinder: **Duembgen, Gerd, Dr.**
**Auerstrasse 14**
**D-6700 Ludwigshafen(DE)**

(72) Erfinder: **Fouquet, Gerd, Dr.**
**Maxburgstrasse 2**
**D-6730 Neustadt(DE)**

(72) Erfinder: **Krabetz, Richard, Dr.**
**Unterer Waldweg 8**
**D-6719 Kirchheim(DE)**

(72) Erfinder: **Merger, Franz, Dr.**
**Max-Slevogt-Strasse 25**
**D-6710 Frankenthal(DE)**

(72) Erfinder: **Nees, Friedbert, Dr.**
**Ringstrasse 25**
**D-7515 Linkenheim-Hochstetten(DE)**

(72) Erfinder: **Vogel, Herbert, Dr.**
**Budapester Strasse 51**
**D-6700 Ludwigshafen(DE)**

## (54) Verfahren zur Herstellung von Methacrylsäure.

(57) Die Erfindung betrifft ein Verfahren zur Herstellung von Methacrylsäure durch Gasphasenoxidation von Methacrolein, das durch Umsetzung von Propanal mit Formaldehyd gewonnen ist, mit Sauerstoff-enthaltenden Gasen in Gegenwart von Wasserdampf an Molybdän und Phosphor in oxidischer Form enthaltenden Katalysatoren, bei dem die Gasmischung, bezogen auf das Methacrolein, weniger als 0,2 Gew.% an Sauerstoff-haltigen und/oder ungesättigten organischen Verbindungen mit mehr als 4 Kohlenstoffatomen enthält.

EP 0 194 620 A2

Croydon Printing Company Ltd.

Verfahren zur Herstellung von Methacrylsäure

Es ist bekannt, Methacrylsäure durch Gasphasenoxidation von Methacrolein mit Sauerstoff-enthaltenden Gasgemischen in Gegenwart von Wasserdampf an Molybdän- und Phosphor-haltigen Katalysatoren herzustellen. Das umzusetzende Methacrolein wird bei technischen Verfahren durch Gasphasenoxidation von Isobuten oder tert.-Butanol gewonnen.

Bei einem anderen, in der DE-PS 875 194 beschriebenen Verfahren, wird Methacrolein durch Umsetzung von Propanal mit Formaldehyd in Gegenwart von primären oder sekundären Aminen in flüssiger Phase bei Temperaturen von über 40°C hergestellt.

Bei der Weiterverarbeitung des nach dem Verfahren der letztgenannten Art durch Kondensation von Propanal und Formaldehyd gewonnenen Methacroleins zu Methacrylsäure durch katalytische Gasphasenoxidation an Mo- und P-haltigen Katalysatoren werden jedoch in der Praxis schlecht reproduzierbare Ergebnisse hinsichtlich des Umsatzes, der Ausbeuten und der Lebensdauer des Katalysators erhalten. Es besteht daher ein großes Interesse, den Umsatz und die Ausbeuten reproduzierbar zu verbessern, da die Herstellung von Methacrylsäure aus Propanal und Formaldehyd und damit letzten Endes aus Ethylen und Synthesegas sowohl verfahrenstechnische als auch wirtschaftliche Vorteile gegenüber Verfahren z.B. auf Basis Isobutylen verspricht.

Es wurde nun gefunden, daß man Methacrylsäure durch Gasphasenoxidation von Methacrolein, das durch Umsetzung von Propanal mit Formaldehyd gewonnen ist, mit Sauerstoff-enthaltenden Gasen in Gegenwart von Wasserdampf an Molybdän- und Phosphor enthaltenden Katalysatoren mit Vorteil herstellen kann, wenn die Gasmischung bezogen auf das Methacrolein weniger als 0,2 Gew.% an Sauerstoff-haltigen und/oder ungesättigten organischen Verbindungen mit mehr als 4 Kohlenstoffatomen enthält.

Bei der Synthese des Methacroleins aus Propanal und Formaldehyd werden in geringer Menge Sauerstoff-haltige und/oder ungesättigte Nebenprodukte mit mehr als 4 Kohlenstoffatomen wie 2-Methylpentenal, dimeres Methacrolein, 2-Methylpentanal, 3-Methoxy-isobutyraldehyd und manchmal Trimethylolethan, Mono-, Di- und Triethyltrioxan gebildet, die die Leistung der Molybdän und Phosphor enthaltenden Oxidationskatalysatoren schon in sehr geringen Konzentrationen stark herabsetzen und zeitaufwendige sowie die Katalysatorlebensdauer verkürzende regenerative Maßnahmen erfordern.

Der Mechanismus der Einwirkung der höhermolekularen Verbindungen auf den Katalysator ist zwar noch nicht völlig geklärt, und nicht alle störenden

höhermolekularen Nebenprodukte der Methacroleinsynthese sind identifiziert; es hat sich jedoch gezeigt, daß vor allem das 2-Methyl-2-pentenal (I) und das dimere Methacrolein (II)

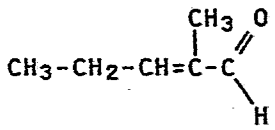
I

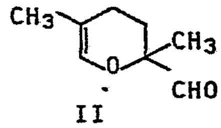
II

die Katalysatorleistung herabsetzen. Es wird angenommen, daß diese beiden Verbindungen auf der Katalysatoroberfläche mit hoher Adsorptionsenergie festgehalten werden und diese für die Oxidation des Methacroleins blockieren. Es wurde gefunden, daß die Konzentration der beiden Verbindungen (I) und (II) jeweils weniger als 0,15 Gew.% (bezogen auf Methacrolein) betragen soll. Vorteilhaft beträgt die Konzentration an (I) und (II) weniger als 0,10 Gew.%. Die Summe beider Verbindungen soll im allgemeinen 0,15 Gew.%, insbesondere 0,1 Gew.% nicht übersteigen. Die Nebenprodukte der genannten Art können von dem Methacrolein in an sich üblicher Weise z.B. durch Absorption oder Destillation abgetrennt werden.

Als Katalysatoren sind die an sich für die Methacroleinoxidation bekannten Phosphor- und Molybdän-haltigen oxidischen Katalysatoren geeignet, die sich von den Phosphor-haltigen Heteropolysäuren des Mo, V, W und deren Alkali- und Erdalkalisalzen ableiten.

Derartige Katalysatoren sind z.B. aus der US-PS 4 409 128 sowie der GB-PS 2 133 307 bekannt.

Sie werden im allgemeinen hergestellt, indem man Oxide oder Verbindungen der Komponenten in wäßrigen oder Wasser-freien organischen Medien unter Bedingungen zusammengibt, die zur Bildung der Phosphor-haltigen Heteropolysäuren des Molybdäns, Vanadins, Wolframs oder ihrer Salze führen. Das Produkt wird dann getrocknet, verformt und schließlich vorzugsweise durch Calcinierung bei höheren Temperaturen aktiviert.

Bei der Gasphasenoxidation des Methacroleins werden als Oxidationsmittel Sauerstoff-haltige Gasmischungen eingesetzt, die neben Stickstoff und gegebenenfalls Kohlenoxiden vorteilhaft Wasserdampf enthalten. Das Verfahren wird im allgemeinen unter einem Druck von 1 bis 5 bar, vorzugsweise 1 bis 2,5 bar, bei einer Verweilzeit von 0,5 bis 5 sec, vorzugsweise von 1 bis 3 sec und bei Temperaturen von 200 bis 370$^{0}$C, vorzugsweise 250 bis 340$^{0}$C durchgeführt.

Im Synthesegas beträgt das molare Verhältnis Methacrolein : Wasserdampf : Sauerstoff : Inertgas im allgemeinen 1 : 1 bis 20 : 1 bis 6 : 4 bis 50, vorzugsweise 1 : 2 bis 10 : 1,55 bis 4 : 6 bis 30.

Die Umsetzung des Methacroleins wird häufig in Rohrbündelreaktoren durchgeführt, in denen der Katalysator fest angeordnet ist. Man kann aber auch in einer Wirbelschicht arbeiten. Fest angeordnete Katalysatorschüttungen werden bevorzugt.

Das Reaktorabgas wird im allgemeinen, gegebenenfalls nach indirekter Kühlung auf 180 bis 250°C, bei Temperaturen zwischen 40 und 80°C mit Wasser gewaschen, wobei wäßrige Lösungen der Methacrylsäure erhalten werden, die zusätzlich kleine Menge an Essig-, Acryl-, Malein-, Fumar-, Citracon- und Ameisensäure, Aldehyde und Ketone sowie, je nach den Reaktions- und Waschbedingungen, mehr oder weniger nicht umgesetztes Methacrolein enthalten können. Die Methacrylsäure wird im allgemeinen aus der wäßrigen Lösung mit organischen Lösungsmitteln extrahiert und destillativ vom Extraktionsmittel und den Nebenprodukten abgetrennt. Das nicht umgesetzte Methacrolein kann zum Teil aus dem wäßrigen Kondensat destillativ oder durch Strippen mit Inertgasen abgetrennt, z.T. aus dem Abgas der Waschkolonne mit Wasser bei niedrigen Temperaturen ausgewaschen und wieder in die Synthese zurückgeführt werden, vorzugsweise nach Verdampfen zusammen mit einem Teil des Abgases der Waschkolonne.

Das eingesetzte Methacrolein, das nach dem Verfahren der Kondensation von Propanal mit Formaldehyd gewonnen wird, enthält im allgemeinen neben den ungesättigten und/oder Sauerstoff-haltigen organischen Verbindungen mit mehr als 4 C-Atomen Wasser in Konzentrationen von ca. 0,5 bis 3 Gew.% noch geringe Mengen an Methanol, Formaldehyd und Propionaldehyd. Die Konzentrationen des Formaldehyds und Propionaldehyds sollen im allgemeinen, bezogen auf Methacrolein, 1 Gew.%, vorzugsweise 0,5 Gew.%, nicht überschreiten, um unerwünschte Nebenreaktionen zu vermeiden.

Die in den folgenden Beispielen angegebenen Teile und Prozente beziehen sich, wenn nichts anderes angegeben, auf das Gewicht. Die Volumenteilen verhalten sich zu den Gewichtsteilen wie das Liter zum Kilogramm.

Beispiel 1

212 Teile Ammoniumheptamolybdat, 22,6 Teile Phosphorsäure (85 %ig), 11,7 Teile Ammoniummetavanadat, 39 Teile Caesiumnitrat, 12,08 Teile Kupfernitrat und 2 Teile Rhodium(III)chlorid-hydrat werden nacheinander unter Rühren in 1200 Teilen Wasser bei 60°C gelöst. Die erhaltene Suspension wird auf dem Wasserbad eingedickt und der Rückstand anschließend

12 Stunden bei 130°C getrocknet. Die Trockenmasse wird zerkleinert und nach Zusatz von 2 Gew.% Graphitpulver zu 5 x 3 mm Tabletten verpreßt.

1,1 Volumenteile der Katalysatortabletten werden in ein Reaktionsrohr von 26 mm innerem Durchmesser, das mit einem axial geführten Temperaturmeßrohr von 8 mm Außendurchmesser versehen ist und das von einem Salzbad beheizt wird, gefüllt. In das Reaktionsrohr leitet man bei einer Badtemperatur von 318°C stündlich 1 103 Volumenteile (bei Normalbedingungen) einer Gasmischung aus 6 Vol.% Methacrolein, 8,8 Vol.% Sauerstoff, 25,2 Vol.% Wasserdampf und 60 Vol.% Stickstoff.

Das eingesetzte Methacrolein wird aus einer gekühlten Vorlage einem mit 14 bar-Dampf beheizten Sprühverdampfer zugeführt und dort in das vorgeheizte Gemisch der Restgase verdampft. Das Methacrolein enthält 0,08 % eines Gemisches an 2-Methylpentenal (2-MP) und 0,02 % seines Gewichts an dimerem Methacrolein (DIM) neben 0,42 % seines Gewichts Propanal und 0,3 % seines Gewichts Formaldehyd.

Aus den Analysenergebnissen der Reaktorausträge errechnet sich ein Methacroleinumsatz von 57,2 Mol.%, eine Ausbeute an Methacrylsäure von 50,8 Mol.% und eine Selektivität der Methacrylsäurebildung von 88,8 Mol.%.

Vergleichsbeispiel 1a

Wird unter den Bedingungen des Beispiels 1 ein Methacrolein mit einem Gehalt von 0,18 Gew.% 2-MP und 0,42 Gew.% DIM eingesetzt, so werden die in der Tabelle 1 zusammengefaßten Ergebnisse erzielt. Innerhalb der Testzeit von 4 Tagen nimmt der Umsatz und die Ausbeute stetig ab.

Tabelle 1

| Zusatz 2-MP | DIM (Gew.%) | zusammen | Testzeit (Tage) | Umsatz (Mol.%) | Ausbeute (Mol.%) |
|---|---|---|---|---|---|
| 0,18 | 0,42 | 0,60 | 0 | 55,9 | 49,5 |
| | | | 1 | 54,6 | 48,8 |
| | | | 2 | 52,1 | 46,5 |
| | | | 3 | 50,6 | 45,2 |
| | | | 4 | 50,6 | 44,6 |

Vergleichsbeispiel 1b

Beispiel 1 wird wiederholt mit der Änderung, das das eingesetzte Methacrolein zusammen 0,21 Gew.% MP und DIM, 1,32 Gew.% Propanal und 0,3 Gew.% Formaldehyd enthält. Unter den Testbedingungen des Beispiels 1 beträgt der Umsatz 53,7 Mol.% und die Ausbeute 48,3 Mol.%.

Vergleichsbeispiele 1c bis 1e

Man arbeitet im wesentlichen wie unter Beispiel 1 angegeben, verändert aber den Gehalt des Methacroleins an 2-MP und DIM.

Die Ergebnisse sind in Tabelle 2 zusammengestellt.

Beispiel 2 und Vergleichsbeispiele 2a bis 2b

Man arbeitet im wesentlichen wie in Beispiel 1 angegeben, verändert aber den Gehalt des Methacrolein an DIM. Die Ergebnisse sind in der folgenden Tabelle 2 zusammengestellt.

Tabelle 2

| | Zusatz 2-MP | DIM (Gew.%) | zusammen | Testzeit (Tage) | Umsatz | Ausbeute (Mol.%) |
|---|---|---|---|---|---|---|
| Beispiel 1 | 0,08 | 0,02 | 0,10 | 1 | 57,2 | 50,8 |
| Vergleichsbeispiel 1c | 0,12 | 0,03 | 0,15 | 2 | 53,7 | 48,1 |
| 1d | 0,30 | 0,03 | 0,33 | 4 | 53,0 | 47,0 |
| 1e | 0,42 | 0,09 | 0,51 | 4 | 48,1 | 43,0 |
| Beispiel 2 | 0,06 | 0,04 | 0,10 | 2 | 56,5 | 51,0 |
| Vergleichsbeispiel 2a | 0,09 | 0,40 | 0,49 | 3 | 50,6 | 45,3 |
| 2b | 0,22 | 0,45 | 0,67 | 2 | 46,9 | 42,7 |

Beispiel 3 und Vergleichsbeispiele 3a bis 3b

300 Teile $MoO_3$, 18,9 Teile $V_2O_5$ (99 %ig), 24 Teile Phosphorsäure (85 %ig), 5,4 Teile $As_2O_5 \cdot H_2O$ und 3,3 Teile CuO werden in 3 000 Teilen

Wasser eingetragen und 3 Stunden unter Rückfluß gekocht. Nach Zugabe von 1,35 Teilen KOH (85 %ig) gelöst in 30 Teilen Wasser wird die Lösung weitere 3 Stunden unter Rückfluß zum Sieden erhitzt. Anschließend wird auf dem Wasserbad eingedampft und 12 Stunden bei 85°C nachgetrocknet. Das Produkt wird zerkleinert, mit 2 Gew.% Graphitpulver vermischt und zu 5 x 3 mm Tabletten verpreßt.

1 Volumenteil des erhaltenen Katalysators wird in den in Beispiel 1 angegebenen Salzbadreaktor gefüllt und darüber bei einer Badtemperatur von 327°C stündlich 1 590 Normalvolumenteile einer Gasmischung aus 4,5 Vol.% Methacrolein, 10 Vol.% Sauerstoff, 22,5 Vol.% Wasserdampf und 63 Vol.% Stickstoff geleitet.

In drei Versuchsreihen wird Methacrolein mit unterschiedlichem Reinheitsgrad hinsichtlich der höhermolekularen Nebenprodukte 2-MP und DIM eingesetzt. Die Ergebnisse sind in der folgenden Tabelle 3 zusammengestellt und zeigen den stark hemmenden Einfluß der Nebenprodukte auf den Umsatz und die Ausbeute.

Tabelle 3

| | Zusatz 2-MP | DIM (Gew.%) | zusammen | Testzeit (Tage) | Umsatz | Ausbeute (Mol.%) |
|---|---|---|---|---|---|---|
| Beispiel 3 | 0,05 | 0,03 | 0,08 | 2 | 63,7 | 50,5 |
| Vergleichsbeispiel 3a | 0,40 | 0,39 | 0,79 | 2 | 57,8 | 45,8 |
| 3b | 0,11 | 1,26 | 1,37 | 2 | 54,3 | 44,1 |
| | | | | 4 | 50,5 | 40,4 |

Patentansprüche

1. Verfahren zur Herstellung von Methacrylsäure durch Gasphasenoxidation von Methacrolein, das durch Umsetzung von Propanal mit Formaldehyd gewonnen ist, mit Sauerstoff-enthaltenden Gasen in Gegenwart von Wasserdampf an Molybdän und Phosphor in oxidischer Form enthaltenden Katalysatoren, dadurch gekennzeichnet, daß die Gasmischung, bezogen auf das Methacrolein, weniger als 0,2 Gew.% an Sauerstoff-haltigen und/oder ungesättigten organischen Verbindungen mit mehr als 4 Kohlenstoffatomen enthält.

2. Verfahren nach Anspruch 1, dadurch gekennzeichnet, daß die Summe der Konzentrationen an 2-Methylpentenal und dimerem Methacrolein, bezogen auf Methacrolein, weniger als 0,15 Gew.% beträgt.

3. Verfahren nach Anspruch 1 bis 2, dadurch gekennzeichnet, daß die Konzentration des Propanals und Formaldehyds, bezogen auf Methacrolein, weniger als 1,0 Gew.% beträgt.